# EUROPEAN PATENT APPLICATION

(11) **EP 4 706 564 A1**
(43) Date of publication of application: **11.03.2026**
(21) Application number: 24199520.8
(22) Date of filing: 10.09.2024
(51) Int. Cl.: A61B 17/34, H01F 13/00

(54) **METHOD AND APPARATUS FOR PROCESSING AN ELONGATED BODY FOR A MEDICAL INSTRUMENT**

(71) Applicant: eZono AG, 07743 Jena (DE)
(72) Inventor: The designation of the inventor has not yet been filed
(74) Representative: J A Kemp LLP

(57) **Abstract**

A disclosure relates to methods and apparatus for processing an elongated body for a medical instrument. In one arrangement, targeted processing is applied to each of one or more local portions of the elongated body to cause a change in a room temperature magnetic property of material in each local portion, thereby causing the one or more local portions to have different room temperature magnetic properties than other portions of the elongated body.

## Description

The present disclosure relates to methods and apparatus for processing an elongated body for a medical instrument.

Many medical procedures require insertion of an elongated medical instrument into a subject's body and/or manipulation of the elongated medical instrument within the body. Examples of such medical instruments include needles, cannulas, catheters and stylets. Examples of such medical procedures include minimally-invasive surgical procedures, local anaesthesia, detection of bioelectrical signals, electrical stimulation for diagnosis or treatment, vascular access, fine needle aspiration, and musculoskeletal injections. The procedures may require accurate guiding of the instrument to a desired position in the subject's body. However, it can be challenging to monitor the position and/or orientation of the instrument during movement of the instrument inside the patient's body.

Ultrasound imaging has been used to help guiding needle and catheterisation procedures. However, for different technical and handling reasons, it is very difficult to track needles in ultrasound images. Practitioners may also rely on their skill and experience to judge positioning of medical instruments based on feel, but this requires extensive training and is prone to human error.

Magnetic tracking of medical instruments has been proposed as an alternative approach. A magnetometric detector may, for example, be attached to a freehand ultrasound probe and used to detect the position and/or orientation of a magnetised tissue-penetrating tool. According to this approach, a freehand ultrasound probe may comprise an array of magnetometric sensors that detect the magnetic field from the magnetised tissue-penetrating tool. A base station may then calculate a relative position and orientation of the tissue-penetrating medical tool relative to the ultrasound probe based on measurements performed by the sensors. The base station can supply this calculated position and orientation to the ultrasound imaging system so that the tissue-penetrating medical tool can be displayed on the ultrasound image of the subject's anatomy.

The system is advantageous in that it allows the operator to see both the ultrasound imaged anatomy and the magnetically detected tissue-penetrating medical tool on the same image. This enables greater accuracy in the procedure. Further, the attachment of a magnetometric detector to the ultrasound probe does not alter the feel of the ultrasound probe significantly, and it remains, therefore, familiar to the practitioner. Similarly the magnetization of the tissue-penetrating medical tool does not alter its mechanical characteristics, which again preserves familiarity for the clinician and enables best use to be made of previous experience. Because the ultrasound probe can be manipulated freely, the ease-of-use of the freehand ultrasound system is also preserved.

It can be challenging to provide magnetised tissue-penetrating tools that reliably have an optimal magnetization at the point of use. Magnetization as a single dipole may not be adequate for tissue-penetrating tools that are relatively long. Magnetization with multiple dipoles may be complex to implement, particularly at a point of use. Magnetization applied during initial manufacture may be prone to being disturbed between the point of manufacture and the point of use.

It is an object of the invention to provide methods and apparatus supporting the provision of improved medical instruments for use in surgical operations that involve magnetic detection of the position and/or orientation of the medical instruments.

According to an aspect of the disclosure, there is provided a method of processing an elongated body for a medical instrument, comprising: applying targeted processing to each of one or more local portions of the elongated body to cause a change in a room temperature magnetic property of material in each local portion, thereby causing the one or more local portions to have different room temperature magnetic properties than other portions of the elongated body, wherein room temperature is defined as 298K.

Thus, a method is provided that allows room temperature magnetic properties of an elongated body to be selectively changed in one or more local portions of the elongated body. This facilitates provision of a pattern of magnetization to the elongated body that is more complex that a single dipole along the whole length of the elongated body. For example, the method facilitates provision of a pattern of magnetization that consists of a dipole that is more localized than a dipole that extends along the whole length of the elongated body (e.g., in the case where only a single local portion is processed) or a pattern of magnetization that comprises multiple separate (e.g., spatially separated) dipoles along the length of the elongated body. Such patterns of magnetization may allow better monitoring of the position of the elongated body during use in a medical procedure, for example via a magnetometric detector. In contrast to alternative techniques that require complex localized application of different magnetic fields to different sections of an elongated body, the present method allows desirable patterns of magnetization to be achieved via application of a uniform magnetic field to the whole elongated body, which is much more convenient and requires simpler and/or less expensive and/or less bulky apparatus. For example, a simple single dipole magnetizer can be used to provide relatively complex patterns of magnetization in the elongated body.

Optionally, the material of the elongated body before the targeted processing is ferromagnetic at room temperature or ferrimagnetic at room temperature. Optionally, the change in the room temperature magnetic property comprises a reduction in the maximum potential residual magnetization that the material is capable of retaining in the absence of an external magnetic field. Thus, magnetizability is selectively suppressed in the selected portions of the elongated body, such that subsequent application of an external magnetic field, even a uniform external magnetic field such as from a simple dipole magnetizer, will achieve desired pattern of magnetization that is more than simply a single dipole extending along the whole length of the elongated body.

According to an aspect of the disclosure, there is provided an apparatus for processing an elongated body for a medical instrument, comprising: a holder for holding the elongated body; and a processing module configured to apply targeted processing to each of one or more local portions of the elongated body to cause a change in a room temperature magnetic property of material in each local portion, thereby causing the one or more local portions to have different room temperature magnetic properties than other portions of the elongated body, wherein room temperature is defined as 298K.

Embodiments of the disclosure will be further described by way of example only with reference to the accompanying drawings.

Figure 1 is a schematic side view of an apparatus for processing an elongated body for a medical instrument.

Figure 2 is a schematic end sectional view of a processing module of the apparatus of Figure 1 with an elongated body positioned for processing by the processing module.

Figure 3 is a schematic end sectional view of the elongated body after processing by the processing module of Figure 2.

The present disclosure relates to methods and apparatus for providing elongated bodies for use as medical instruments or portions of medical instruments.

References are made below to room temperature in relation to describing magnetic properties of materials, which typically vary as a function of temperature. Room temperature is understood in this context to be 298K, although in practice the magnetic properties in question will be substantially the same through a range of temperatures at which medical instruments may conceivably be used, such as in a range of about 30K above and below 298K. The magnetic properties in question will also typically be substantially the same through a broader range of temperatures that goes beyond those at which medical instruments may conceivably be used during an operation, including temperatures that may be used for sterilization prior to an operation.

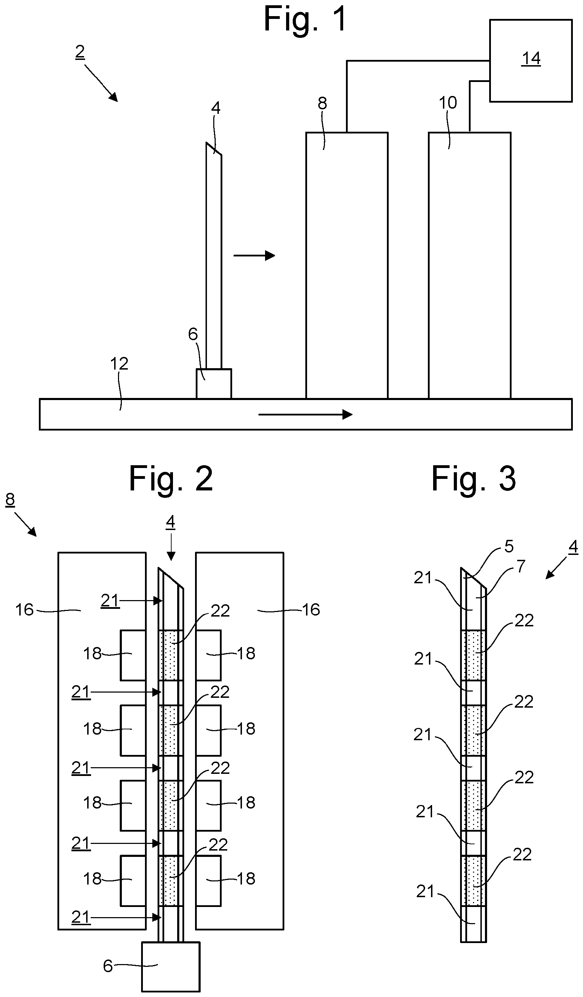

Figure 1 depicts an example apparatus 2 for performing a method of processing an elongated body 4 for a medical instrument according to the disclosure. The method may be used for processing a wide range of elongated bodies 4 for medical instruments and is not limited to any particular type. The method is particularly applicable to tissue-penetrating tools. The elongated body 4 may comprise a portion or all of one or more of the following: a needle, a cannula, a stylet, a guidewire, and a cable. The elongated body 4 may therefore take various forms, including one or more of the following: a solid elongated body (cylindrical or otherwise); a hollow elongated body (cylindrical or otherwise); a composite structure comprising a core wire and a coiled wire around the core wire (e.g., to form a guidewire); and a solid or elongated body (cylindrical or otherwise) with one or more longitudinal recesses (which may be referred to as channels) running along a portion or all of the length of the body. In the examples discussed below, the elongated body 4 is a hollow cannula comprising a cylindrical body 5 defining an inner lumen 7, but the apparatus and methods described could equally be used with any other suitable type of elongated body 4 for a medical instrument, including any of those listed above.

In the example of Figure 1, the apparatus 2 comprises a holder 6 for holding the elongated body 4. The holder 6 may, for example, comprise a gripping mechanism for gripping an elongated body 4 or a plurality of elongated bodies 4. The gripping mechanism may be adjustable to allow the elongated body or bodies to be selectively clamped (e.g., clamped for processing and unclamped for insertion and/or removal) or may grip by friction fitting or any other suitable holding approach. In the example shown, the holder 6 holds an elongated body 4 vertical (i.e., with an axis of elongation of the elongated body 4 aligned vertically), but this is not essential. The holder 6 may in general hold the elongated body or bodies so that they are aligned in any convenient direction.

The apparatus 2 further comprises a processing module 8. The processing module 8 is configured to apply targeted processing to each of one or more local portions 22 of the elongated body 4. The one or more local portions 22 may, for example, comprise a single portion, two portions, three portions, four portions, five portions, or more than five portions. Each local portion 22 may comprise a portion of a length of the elongated body 4. Each local portion 22 may be separated from each other local portion 22. For example, each local portion 22 may comprise a different portion of the length of the elongated body 4. Examples of local portions 22 are depicted in Figures 2 and 3. Portions of the elongated body 4 other than the local portions 22 may be referred to as other portions 21 or unprocessed portions 21. The targeted processing is targeted in a spatial sense. One or more local portions 22 are processed by the targeted processing while one or more other portions 21, in different spatial locations, are substantially not processed or processed significantly less.

The targeted processing performed by the processing module 8 is such as to cause a change in a room temperature magnetic property of material in each local portion 22. As mentioned above, magnetic properties of materials may vary as a function of temperature. For example, ferromagnets may undergo a phase transformation to a paramagnetic state when heated above the Curie transition temperature but will typically transition back to being ferromagnetic again when cooled below the Curie transition temperature. The room temperature magnetic property of the material refers to the magnetic property of the material when the material is at room temperature.

The change in the room temperature magnetic property caused by the targeted processing causes the one or more local portions 22 to have different room temperature magnetic properties than other portions 21 of the elongated body 4. The targeted processing may for example change the magnetic properties of the local portions 22 without significantly changing the magnetic properties of other portions 21 of the elongated body 4 or may change the magnetic properties of the local portions 22 in a way that is different (e.g., stronger) than any change applied to the other portions 21 of the elongated body 4.

The material of the elongated body 4 will typically have the same room temperature magnetic properties along the whole length of the elongated body 4 before the targeted processing is applied to the elongated body 4. The elongated body 4 may, for example, be supplied as a single integral body of substantially uniform composition and/or internal structure and/or phase (e.g., austenite phase or ferrite phase of a stainless steel) and/or mixture of phases (e.g., proportion of austenite phase relative to ferrite phase of a stainless steel).

The material of the elongated body 4 before the targeted processing is typically ferromagnetic or ferrimagnetic at room temperature. Thus, the material of the elongate body 4 can retain a net magnetization at room temperature without an applied external magnetic field being present. The elongate body 4 may have a range of compositions. The elongate body 4 may, for example, comprise a metallic alloy. In some implementations, the elongate body 4 comprises a ferromagnetic stainless steel, such as a ferritic stainless steel, a martensitic stainless steel, or precipitation hardenable stainless steel. Stainless steel grades 304, 306, 316 and/or 17-7 may, for example, be used.

The change in the room temperature magnetic property of material in each local portion 22 caused by the targeted processing may comprise a reduction in the maximum residual magnetization that the material can retain in the absence of an external magnetic field. The reduction may be by at least 50%, optionally by at least 75%, optionally by at least 90%, optionally by at least 95%. The residual magnetization may also be referred to as residual magnetism, remanence, remanent magnetization, or retentivity.

In some implementations, the change in the room temperature magnetic property comprises reducing the maximum potential residual magnetization to substantially zero. Thus, the targeted processing may for example cause the one or more local portions 22 to change from being ferromagnets (or ferrimagnets) at room temperature to being paramagnets at room temperature.

Thus, when the elongated body 4 is ferromagnetic or ferrimagnetic before the targeted processing, applying an external magnetic field to the elongated body 4 after the targeted processing will lead to a non-uniform pattern of magnetization along the elongated body 4 that is defined by the locations of the one or more local portions 22 whose magnetic properties have been changed by the targeted processing. The non-uniform pattern may, for example, comprise a relatively strong magnetic dipole in each unprocessed portion 21 of the elongate body 4 and no dipole (or a weaker dipole) in each of the one or more processed local portions 22.

In some implementations, the change in the room temperature magnetic property comprises an increase in the maximum potential residual magnetization of the material. This variation may be useful for example where the material of the elongate body 4 before the targeted processing is not ferromagnetic at room temperature, is not ferrimagnetic at room temperature, or only has a relatively low maximum potential residual magnetization. In this case, applying an external magnetic field to the elongated body 4 after the targeted processing will again lead to a non-uniform pattern of magnetization along the elongated body 4 that is defined by the locations of the one or more local portions 22 whose magnetic properties have been changed by the localized processing. In this case, however, the non-uniform pattern may comprise no dipole (or a relatively weak dipole) in each unprocessed portion 21 of the elongated body 4 and a stronger magnetic dipole in each of the processed local portions 22.

In the example apparatus 2 shown in Figure 1, the apparatus 2 further comprises a magnetizer 10. The magnetizer 10 is configured to apply an external magnetic field to the elongated body 4. The external magnetic field is typically such that substantially all of the elongated body 4 experiences a magnetic field strength that is above a threshold value that is suitable for magnetizing the material of the elongated body 4 before the elongated body 4 has been processed by the processing module 8. The magnetic field strength may, for example, be high enough to magnetize the elongated body 4 to the maximum extent possible, for example into a saturated state. The external magnetic field may be applied substantially uniformly to at least a majority of the elongated body 4. Optionally, the external magnetic field is applied substantially uniformly to all of the elongated body 4.

The external magnetic field may be such that if applied to an elongated body 4 that has not been subjected to the targeted processing, the magnetic field would magnetize the elongated body 4 into a single dipole. The external magnetic field may be such that if applied to an elongated body 4 that has been subjected to the targeted processing, the magnetic field would induce a dipole magnetization in each of the one or more local portions 22 of the elongated body 4, and induce no dipole magnetization, or only a weaker dipole magnetization, in portions 21 of the elongated body 4 other than the one or more local portions 22. Thus, the external magnetic field magnetizes the one or more local portions 22 of the elongated body 4 less than the rest of the elongated body 4 or substantially does not magnetize the one or more local portions 22 of the elongated body 4.

The example apparatus 2 shown in Figure 1 is configured to implement embodiments of the method in which the external magnetic field is applied after the targeted processing. However, this is not essential. In some implementations of the method, the external magnetic field is applied before the targeted processing. The external magnetic field may magnetize the whole elongated body 4, for example into a single dipole. The targeted processing may cause at least partial demagnetization of the one or more local portions 22 of the elongated body 4, thereby defining a non-uniform pattern of magnetization along the elongated body 4 that is defined by the locations of the one or more local portions 22 whose magnetic properties have been changed by the localized processing.

In some implementations, the targeted processing comprises a heat treatment. The heat treatment comprises selective heating of the one or more local portions 22 of the elongated body 4. The selective heating may involve raising the temperature of the one or more local portions 22 above a reference temperature. The reference temperature may be equal to or higher than 500 degrees C, optionally equal to or higher than 600 degrees C, optionally equal to or higher than 700 degrees C, optionally equal to or higher than 750 degrees C, optionally equal to or higher than 800 degrees C. The selectively heating may involve maintaining the temperature of the one or more local portions 22 above the reference temperature for a predetermined time. The predetermined time may be relatively short to avoid excessive conduction of heat to one or more other portions 21 of the elongated body 4 adjacent to the one or more local portions 22. The predetermined time may, for example, be less than about 30 seconds, optionally less than about 20 seconds, optionally less than about 10 seconds, optionally less than about 5 seconds. After the heat treatment, the elongated body 4 may be allowed to cool by stopping application of any heating and allowing the elongated body 4 to cool to room temperature under ambient conditions. Alternatively, the elongated body 4 may be cooled more quickly (quenched) if useful for achieving the desired change in magnetic properties for a particular choice of composition of the elongated body 4.

For some compositions of elongated body 4, the heat treatment may be such that a proportion of the material of the elongated body 4 that is in a phase that is non-ferromagnetic (e.g., paramagnetic) at room temperature (e.g., an austenite phase) is higher in the one or more local portions 22 of the elongated body 4 after the heat treatment than before the heat treatment. For example, the elongated body 4 may be formed from a stainless steel that can exist in a ferromagnetic phase (e.g., a ferrite phase) and a paramagnetic phase (e.g., an austenite phase) and the targeted processing may be such as to increase an amount of austenite phase that is present at room temperature relative to an amount of ferrite phase that is present at room temperature, optionally such that there is substantially no ferrite phase present at room temperature.

Figure 2 depicts an example processing module 8 for performing the targeted processing of local portions 22 of an example elongated body 4 by heat treatment. Figure 3 depicts the elongated body 4 of Figure 2 after the target processing has been completed. Various different approaches may be used to implement the targeted processing. In the example of Figure 2, the processing module 8 comprises a plurality of heaters 18 mounted within a thermally insulating body 16. The heaters 18 apply heating to respective local portions 22 of the elongated body 4 to be processed substantially without causing significant heating to other portions 21 outside of (e.g., between) the local portions 22. The heaters 18 may use any suitable mechanism for achieving this. The heaters 18 may apply localized heating using a laser and/or inductive heating, for example.

The magnetic properties of certain materials, such as metallic alloys such as stainless steels, may also be affected by mechanical processing. Thus, alternatively or additionally to the heat treatments described above, the targeted processing may comprise selectively mechanically working the one or more local portions 22 of the elongated body 4. For example, the elongated body 4 could be repeatedly deflected about one or more hinge regions in order to mechanically work the one or more hinge regions and thereby change the magnetic properties of the one or more hinge regions. The one or more hinge regions would then each correspond to a local portion 22 that has been selectively mechanically worked. Subsequent magnetization of the elongated body 4 would lead to a magnetization pattern that is changed by the mechanical working of the one or more local portions 22, for example such that the one or more local portions 22 retain a lower magnetization or substantially no magnetization relative to one or more unprocessed portions 21 of the elongated body 4.

As depicted schematically in Figure 1, the apparatus 2 may further comprise a conveyance arrangement 12. The conveyance arrangement 12 may, for example, comprise a conveyor belt or the like. The conveyance arrangement 12 is configured to bring each of a plurality of elongated bodies 4 in sequence (e.g., one after another) into a position at which the processing module 8 can apply the targeted processing to the elongated body 4 (e.g., aligned with processing module 8 in Figure 1 corresponding to the position of the elongated body 4 depicted in Figure 2) and/or into a position at which the magnetizer 10 can apply the external magnetic field to the elongated body 4 (e.g., aligned with magnetizer 10 in Figure 1). The conveyance arrangement 12 may cooperate with the holder 6, for example by moving the holder 6 in order to move any elongated body or bodies held by the holder 6.

### EXPERIMENTAL RESULTS

The inventors carried out a range of experiments to explore the effects of heating on the magnetic properties of elongated bodies suitable for use as medical instruments.

Preliminary studies showed that heating sections of a stainless steel needle with a flame from a cigarette lighter caused those sections of the needle to have a lower magnetic strength than other sections of the needle after a subsequent magnetization step. A flame from a cigarette lighter typically reaches temperatures in the 900-1000 degrees C. These preliminary studies showed that heating in the range of 900-1000 degrees C can reduce the maximum potential residual magnetization of stainless steel.

Preliminary studies also demonstrated that prolonged exposure of stainless steel needles to a flame from a cigarette lighter leads to the stainless steel becoming brittle.

More detailed experiments were designed based on the preliminary studies to determine the extent to which the magnetic properties of elongated bodies for use as medical instruments can be modified while retaining the mechanical properties that are needed for effective use as medical instruments. These experiments are described below.

As representative examples of elongated bodies for use as medical instruments, 18G 44.5mm long thin-walled cannulas were used. These cannulas were formed from 306 Stainless Steel. The cannulas were grinded on one end. No shaft was added to the cannulas.

The cannulas were magnetized by fully inserting the cannulas into a region containing a uniform strong magnetic field and then removing them. This resulted in each cannular being magnetized as a uniform N-S dipole over the full length of the cannula (43mm).

The magnetization of each cannula was determined by obtaining measurements from magnetic sensors and calculating a best fit to those measurements with a mathematical model of a perfect magnetic dipole that can vary the length and strength to obtain the best fit. Thus, by determining the length and strength of a dipole that provides the best fit to the obtained measurements it is possible to obtain an estimate for the strength of the dipole that is actually present in the cannula being measured. Allowing the length to vary in the optimization was found to provide improved sensitivity for detecting weaker magnetizations.

The experimental process was as follows. A plurality of identical cannulas was split into multiple groups of cannulas. Then for each group the following was carried out:
i) each cannula was magnetized;
ii) the magnetic properties of each cannula were then measured (as described above);
iii) a furnace was pre-heated to a desired temperature for the group;
iv) when the furnace reached the desired temperature, the group of cannulas was placed inside the furnace crucible and the door closed;
v) the cannulas were removed from the furnace after a desired time for the group and then cooled down in air (a normalization process) over a wooden block.
vi) the cannulas were then magnetized again; and
vii) the magnetic properties of each cannula were measured again (as described above).

The above process was repeated for each of six groups of the cannulas. The processing temperatures and times and numbers of cannulas for each group were as follows.

Group 1: 750 degrees C; 30 seconds (1 cannula) and greater than 1 minute (4 cannulas).

Group 2: 750 degrees C; 5 seconds (1 cannula) and greater than 1 minute (4 cannulas).

Group 3: 750 degrees C; 5 seconds (2 cannulas) and greater than 1 minute (3 cannulas).

Group 4: 700 degrees C; 5 seconds (1 cannula) and greater than 1 minute (4 cannulas).

Group 5: 500 degrees C; 5 minutes (1 cannula).

Group 6: Burn single cannula with cigarette lighter flame in two different sections.

Tables 1-5 below show the results of the magnetic measurements before and after the heating process. The tables show the optimized length ("Length") and magnetic strength ("Mag") of the model, together with a residual ("Residual") indicating how good a fit the model is able to achieve to the measurements, for measurements of the magnetic properties before and after the heating. A lower residual indicates a better fit to the measurements.

Magnetization values below 10 indicate negligible magnetic strength. The model finds a solution but it is not within the normal model parameters of length and strength. For these cases we consider the result as "non magnetic cannulas".

**Table 1: Group 1**

| BEFORE | | | AFTER | | | |
|---|---|---|---|---|---|---|
| Length | Mag | Residual | Heating | Length | Mag | Residual |
| 43 | 25 | 40 | 750C 30s | 35 | 2 | 15 |
| 45 | 24 | 40 | 750C > 1min | 32 | 4 | 90 |
| 41.5 | 34 | 40 | 750C > 1min | 35 | 6 | 30 |
| 45.5 | 23 | 40 | 750C > 1min | 18 | 0.5 | 30 |
| 44 | 34 | 40 | 750C > 1min | 40 | 2.5 | |

**Table 2: Group 2**

| BEFORE | | | AFTER | | | |
|---|---|---|---|---|---|---|
| Length | Mag | Residual | Heating | Length | Mag | Residual |
| 43 | 32 | 40 | 750C 5s | 42 | 42 | 20 |
| 43.5 | 30 | 40 | 750C > 1min | 25 | 4 | 20 |
| 44 | 26 | 40 | 750C > 1min | 34 | 4 | 20 |
| 43 | 38 | 40 | 750C > 1min | 30 | 2 | 15 |
| 43 | 28 | 40 | 750C > 1min | 26 | 8 | 20 |

**Table 3: Group 3**

| BEFORE | | | AFTER | | | |
|---|---|---|---|---|---|---|
| Length | Mag | Residual | Heating | Length | Mag | Residual |
| 41.5 | 28 | 40 | 750C 5s | 43 | 34 | 20 |
| 42 | 24 | 40 | 750C 5s | 43 | 27 | 75 |
| 42 | 25 | 40 | 750C > 1min | 30 | 2 | 15 |
| 44 | 26 | 40 | 750C > 1min | 35 | 1 | 15 |
| 43.5 | 29.5 | 40 | 750C > 1min | 0 | 0 | 0 |

**Table 4: Group 4**

| BEFORE | | | AFTER | | | |
|---|---|---|---|---|---|---|
| Length | Mag | Residual | Heating | Length | Mag | Residual |
| 43 | 28 | 40 | 700C 5s | 43.5 | 25 | 20 |
| 42.5 | 37 | 40 | 700C > 1min | 34 | 3 | 25 |
| 43.5 | 26 | 40 | 700C > 1min | 30 | 4 | 60 |
| 42 | 28 | 40 | 700C > 1min | 38 | 4 | 20 |
| 42 | 23 | 40 | 700C > 1min | 32 | 4 | 30 |

**Table 5: Group 5**

| BEFORE | | | AFTER | | | |
|---|---|---|---|---|---|---|
| Length | Mag | Residual | Heating | Length | Mag | Residual |
| | | | 500C 5min | 39 | 20 | 30 |

When heating was applied for longer than one minute at 700 degrees C or higher, a large (almost complete) decrease of magnetization was observed.

At 750 degrees C, a lower heating period of 30 seconds was observed to be sufficient to decrease magnetization to a similar extent as the longer heating periods.

At 500 degrees C, a long heating period of 5 minutes achieved a reduction in magnetization of around 1/2.

The cannulas were mechanically tested following the "ISO 9626:2001 9" standard for "Stainless steel needle tubing for the manufacture of medical devices". The cannulas had a metric size of 1.2 (18G) and are thin-walled. For these characteristics, the bending force to be applied according to the standard is 20N and the maximum deflection is 0.55mm. Cannulas that had not undergone any of the heating processing described above showed a deflection of 0.48mm. Processed cannulas showed a range of deflections from 0.39mm to 0.46mm. All processed cannulas thus became a little stiffer than the unprocessed cannulas but all were also under the 0.55mm deflection limit. The processing thus did not affect the mechanical properties in a way that would prejudice the use of the cannulas as medical devices.

Embodiments of the disclosure are defined by the following numbered clauses.
1. A method of processing an elongated body for a medical instrument, comprising:
   applying targeted processing to each of one or more local portions of the elongated body to cause a change in a room temperature magnetic property of material in each local portion, thereby causing the one or more local portions to have different room temperature magnetic properties than other portions of the elongated body, wherein room temperature is defined as 298K.
2. The method of clause 1, wherein the material of the elongated body has the same room temperature magnetic properties along the whole length of the elongated body before the targeted processing.
3. The method of clause 1 or 2, wherein the elongated body comprises at least a portion of one or more of the following: a tissue-penetrating medical tool, a needle, a cannula, a stylet, a guidewire, a cable.
4. The method of any preceding clause, wherein the elongated body consists of a single integral body of substantially uniform composition before the targeted processing.
5. The method of any preceding clause, wherein the material of the elongated body before the targeted processing is ferromagnetic at room temperature or ferrimagnetic at room temperature.
6. The method of any preceding clause, wherein the change in the room temperature magnetic property comprises a reduction in the maximum potential residual magnetization that the material is capable of retaining in the absence of an external magnetic field, optionally a reduction by at least 50%.
7. The method of clause 6, wherein the change in the room temperature magnetic property comprises reducing the maximum potential residual magnetization to substantially zero.
8. The method of any of clauses 1-5, wherein the change in the room temperature magnetic property comprises an increase in the maximum potential residual magnetization that the material is capable of retaining in the absence of an external magnetic field.
9. The method of any preceding clause, wherein the elongated body comprises a metallic alloy.
10. The method of clause 9, wherein the elongated body comprises a ferromagnetic stainless steel, such as a ferritic stainless steel, a martensitic stainless steel, or precipitation hardenable stainless steel.
11. The method any preceding clause, wherein the targeted processing comprises a heat treatment that comprises selective heating of the one or more local portions of the elongated body.
12. The method of clause 11, wherein the heat treatment is such that a proportion of the material of the elongated body that is in a phase that is non-ferromagnetic at room temperature is higher in the one or more local portions of the elongated body after the heat treatment than before the heat treatment.
13. The method of clause 11 or 12, wherein the selective heating comprises raising the temperature of the one or more local portions of the elongated body above a reference temperature, optionally wherein the reference temperature equal to or higher than 500 degrees C.
14. The method of any preceding clause, wherein the targeted processing comprises selectively mechanically working the one or more local portions of the elongated body.
15. The method of any preceding clause, further comprising applying an external magnetic field to the elongated body, optionally wherein the external magnetic field is applied substantially uniformly to at least a majority of the elongated body.
16. The method of clause 15, wherein the external magnetic field is applied before the targeted processing.
17. The method of clause 16, wherein the external magnetic field magnetizes the whole elongated body.
18. The method of clause 17, wherein the targeted processing causes at least partial demagnetization of the one or more local portions of the elongated body.
19. The method of clause 15, wherein the external magnetic field is applied after the targeted processing.
20. The method of clause 19, wherein the external magnetic field magnetizes the one or more local portions of the elongated body less than the rest of the elongated body or substantially does not magnetize the one or more local portions of the elongated body.
21. The method of any of clauses 15-20, wherein the applying of the external magnetic field is such that:
   if applied to an elongated body that has not been subjected to the targeted processing, the magnetic field would magnetize the elongated body into a single dipole; and
   if applied to an elongated body that has been subjected to the targeted processing, the magnetic field would induce a dipole magnetization in each of the one or more local portions of the elongated body, and induce no dipole magnetization, or only a weaker dipole magnetization, in portions of the elongated body other than the one or more local portions.
22. The method of any preceding clause, wherein the method is applied to each of a plurality of the elongated bodies in sequence, the elongated bodies optionally being conveyed by a conveyor belt.
23. An apparatus for processing an elongated body for a medical instrument, comprising:
   a holder for holding the elongated body; and
   a processing module configured to apply targeted processing to each of one or more local portions of the elongated body to cause a change in a room temperature magnetic property of material in each local portion, thereby causing the one or more local portions to have different room temperature magnetic properties than other portions of the elongated body, wherein room temperature is defined as 298K.
24. The apparatus of clause 23, further comprising a magnetizer configured to apply an external magnetic field to the elongated body, optionally substantially uniformly to at least a majority of the elongated body.
25. The apparatus of clause 23 or 24, wherein the external magnetic field is such that:
   if applied to an elongated body that has not been subjected to the target processing by the processing module, the magnetic field would magnetize the elongated body into a single dipole; and
   if applied to an elongated body that has been subjected to the targeted processing by the processing module, the magnetic field would induce a dipole magnetization in each of the one or more local portions of the elongated body, and induce no dipole magnetization, or only a weaker dipole magnetization, in portions of the elongated body other than the one or more local portions.
26. The apparatus of any of clauses 23-25, further comprising:
   a conveyance arrangement configured to bring each of a plurality of elongated bodies in sequence into a position at which the processing module can apply the targeted processing to the elongated body and/or into a position at which the magnetizer, when present, can apply the external magnetic field to the elongated body.

## Claims

1. A method of processing an elongated body for a medical instrument, comprising:
applying targeted processing to each of one or more local portions of the elongated body to cause a change in a room temperature magnetic property of material in each local portion, thereby causing the one or more local portions to have different room temperature magnetic properties than other portions of the elongated body, wherein room temperature is defined as 298K.

2. The method of claim 1, wherein:
the material of the elongated body has the same room temperature magnetic properties along the whole length of the elongated body before the targeted processing; and/or
the material of the elongated body before the targeted processing is ferromagnetic at room temperature or ferrimagnetic at room temperature.

3. The method of claim 1 or 2, wherein:
the elongated body comprises at least a portion of one or more of the following: a tissue-penetrating medical tool, a needle, a cannula, a stylet, a guidewire, a cable; and/or
the elongated body consists of a single integral body of substantially uniform composition before the targeted processing.

4. The method of any preceding claim, wherein the change in the room temperature magnetic property comprises a reduction in the maximum potential residual magnetization that the material is capable of retaining in the absence of an external magnetic field, optionally a reduction by at least 50%, optionally a reduction to substantially zero.

5. The method of any of claims 1-3, wherein the change in the room temperature magnetic property comprises an increase in the maximum potential residual magnetization that the material is capable of retaining in the absence of an external magnetic field.

6. The method of any preceding claim, wherein the elongated body comprises a metallic alloy, optionally wherein the elongated body comprises a ferromagnetic stainless steel, such as a ferritic stainless steel, a martensitic stainless steel, or precipitation hardenable stainless steel.

7. The method any preceding claim, wherein the targeted processing comprises a heat treatment that comprises selective heating of the one or more local portions of the elongated body, wherein, optionally, the selective heating comprises raising the temperature of the one or more local portions of the elongated body above a reference temperature, optionally wherein the reference temperature equal to or higher than 500 degrees C.

8. The method of claim 7, wherein the heat treatment is such that a proportion of the material of the elongated body that is in a phase that is non-ferromagnetic at room temperature is higher in the one or more local portions of the elongated body after the heat treatment than before the heat treatment.

9. The method of any preceding claim, wherein the targeted processing comprises selectively mechanically working the one or more local portions of the elongated body.

10. The method of any preceding claim, further comprising applying an external magnetic field to the elongated body, optionally wherein the external magnetic field is applied substantially uniformly to at least a majority of the elongated body, wherein:
the external magnetic field is applied before the targeted processing, optionally wherein the external magnetic field magnetizes the whole elongated body, optionally wherein the targeted processing causes at least partial demagnetization of the one or more local portions of the elongated body; or
the external magnetic field is applied after the targeted processing, optionally wherein the external magnetic field magnetizes the one or more local portions of the elongated body less than the rest of the elongated body or substantially does not magnetize the one or more local portions of the elongated body.

11. The method of claim 10, wherein the applying of the external magnetic field is such that:
if applied to an elongated body that has not been subjected to the targeted processing, the magnetic field would magnetize the elongated body into a single dipole; and
if applied to an elongated body that has been subjected to the targeted processing, the magnetic field would induce a dipole magnetization in each of the one or more local portions of the elongated body, and induce no dipole magnetization, or only a weaker dipole magnetization, in portions of the elongated body other than the one or more local portions.

12. The method of any preceding claim, wherein the method is applied to each of a plurality of the elongated bodies in sequence, the elongated bodies optionally being conveyed by a conveyor belt.

13. An apparatus for processing an elongated body for a medical instrument, comprising:
a holder for holding the elongated body; and
a processing module configured to apply targeted processing to each of one or more local portions of the elongated body to cause a change in a room temperature magnetic property of material in each local portion, thereby causing the one or more local portions to have different room temperature magnetic properties than other portions of the elongated body, wherein room temperature is defined as 298K.

14. The apparatus of claim 13, further comprising:
a magnetizer configured to apply an external magnetic field to the elongated body, optionally substantially uniformly to at least a majority of the elongated body; and/or
a conveyance arrangement configured to bring each of a plurality of elongated bodies in sequence into a position at which the processing module can apply the targeted processing to the elongated body and/or into a position at which the magnetizer, when present, can apply the external magnetic field to the elongated body.

15. The apparatus of claim 13 or 14, wherein the external magnetic field is such that:
if applied to an elongated body that has not been subjected to the target processing by the processing module, the magnetic field would magnetize the elongated body into a single dipole; and
if applied to an elongated body that has been subjected to the targeted processing by the processing module, the magnetic field would induce a dipole magnetization in each of the one or more local portions of the elongated body, and induce no dipole magnetization, or only a weaker dipole magnetization, in portions of the elongated body other than the one or more local portions.
